# EUROPEAN PATENT SPECIFICATION

(11) **EP 3 273 942 B1**
(45) Date of publication and mention of the grant of the patent: **16.06.2021**
(21) Application number: 16720351.2
(22) Date of filing: 16.03.2016
(51) Int. Cl.: A61K 9/70, A61K 31/593, A61K 31/661, A61P 17/06

(54) **MICRONEEDLE PATCH FOR DELIVERING AN ACTIVE INGREDIENT TO THE SKIN**
MIKRONADELPFLASTER ZUR ABGABE EINES WIRKSTOFFS IN DIE HAUT
TIMBRE À MICRO-AIGUILLES POUR LA LIBÉRATION D'UN AGENT ACTIF DANS LA PEAU

(30) Priority: 27.03.2015 EP 15161262; 09.07.2015 EP 15176101
(43) Date of publication of application: 31.01.2018
(73) Proprietor: Leo Pharma A/S, 2750 Ballerup (DK)
(72) Inventor: PETERSSON, Karsten, 2750 Ballerup (DK); ENGELL, Karen Margrethe, 2750 Ballerup (DK); JANSSON, Jörgen, 2750 Ballerup (DK); NIELSEN, Kim Troensegaard, 2750 Ballerup (DK); ERIKSSON, André Huss, 2750 Ballerup (DK); MOORE, Anne, Cork (IE); VUCEN, Sonja, Cork (IE); O'SULLIVAN, Caroline, Cork (IE); CREAN, Abina, Cork (IE)
(74) Representative: Leo Pharma A/S
(86) International application number: PCT/EP2016/025026
(87) International publication number: WO 2016/155891

(56) References cited:
- WO-A1-00/64450
- WO-A2-2012/066506
- AU-A1- 2014 200 648
- US-A1- 2009 182 306
- US-A1- 2011 276 028
- US-A1- 2015 057 505

## Description

### FIELD OF INVENTION

The present invention relates to a microneedle patch composition capable of providing sustained release of a therapeutically active ingredient in skin. The composition is intended for use in the treatment of skin conditions.

### BACKGROUND OF THE INVENTION

Human skin, in particular the outer layer, the stratum corneum, provides an effective barrier against penetration into the body of microbial pathogens and toxic chemicals. While this property of the skin is generally beneficial, it complicates the dermal administration of pharmaceuticals in that a significant quantity, if not most, of an active ingredient applied on the skin of a patient suffering from a dermal disease may not penetrate into the viable layers of the skin where it exerts its activity. One way to obtain increased penetration of the active ingredient into the skin is to provide occlusion by formulating the active ingredient in a hydrophobic vehicle such as petrolatum. Penetration into the dermis and epidermis may be boosted by providing the active ingredient in a dissolved state together with a low molecular weight solvent such as ethanol or propylene glycol which may also act as a penetration enhancer and/or by also adding a penetration enhancer to the formulation. However, such measures may not result in adequate penetration, and in addition formulations that contain a high concentration of a hydrophobic excipient, e.g. petrolatum, generally have a tacky or greasy feel that persists for some time after application, and they are consequently considered to be less cosmetically acceptable.

Conventional topical formulations also have to be applied one or more times a day. This is considered an onerous task by many patients who would prefer less frequent dosing and who are therefore more likely to adhere to therapy that involves application every 2 or 3 days or even longer.

Compositions comprising microneedles have been developed as an alternative to transdermal patch formulations to deliver a therapeutically active ingredient or vaccine through skin. Compositions containing microneedles in which an active ingredient is incorporated have also been developed as an alternative to conventional topical formulations such as ointments and creams. Microneedles are micron-scale structures designed to pierce the stratum corneum and permit delivery of an active ingredient transdermally or to the epidermis and dermis. Microneedle arrays have been prepared from many diverse materials such as silicon, stainless steel and biodegradable polymers. One example of a microneedle formulation is solid microneedles coated with a formulation of the active ingredient which is released into the epidermis and dermis when the microneedles have pierced the stratum corneum. Another example of a microneedle formulation is dissolving or biodegradable microneedles prepared from a polymer incorporating the active ingredient which is released gradually as the polymer degrades in the viable layers of the skin.

WO 02/064193 discloses arrays of microneedles composed of polymers and/or metal, for instance a biodegradable polymer such as polylactic acid or polyglycolic acid. The polymer may include a therapeutically active ingredient which is released when the microneedles are inserted into the skin.

WO 2008/130587 discloses arrays of microneedles containing two layers of different polymers, e.g. polyvinyl alcohol and polylactic co-glycolic acid, respectively. One of the layers may contain a therapeutically active ingredient. The other polymer layer is cast on top of the first layer, the solvent is removed, and the microneedle array is removed from the mould. WO 2008/130587 specifically discloses microneedles that comprise a drug-loaded tip composed of a fast dissolving polymer (e.g. polyvinylalcohol) and a base layer of a biodegradable polymer (polylactic co-glycolic acid).

WO 2012/153266 discloses a method of making microneedle arrays by filling microneedle-shaped cavities in a mould with a solvent, applying a microneedle-forming polymer solution on the cavities to mix the solvent and polymer solution by diffusion, removing the solvent and removing the resulting microneedles from the mould.

WO 2012/066506 discloses a method of making microneedles by spraying a composition into a mould, drying the composition and removing the dried composition from the mould.

US 2007/0134829 discloses a method of producing microneedle arrays by wet etching of silicon with a potassium hydroxide solution using a masking material provided with a number of openings for a sufficient period of time to produce microneedles of a specific shape and sharpness. The microneedle arrays may be used for medical applications or as masters to cast moulds for making microneedles of polymeric materials.

It is an object of the invention to provide a topical composition comprising microneedles of a biodegradable polymer with the aim of improving delivery of a therapeutically active ingredient into the viable layers of the skin, in particular the dermis and/or epidermis. It is a further object of the invention to provide a microneedle composition which forms a drug reservoir in the skin from which the active ingredient is released over a prolonged period of time so that the composition may be administered less frequently than conventional topical formulations such as creams or ointments.

### SUMMARY OF THE INVENTION

In the course of research leading to the present invention, it was found possible to provide a microneedle composition with a layered structure that permits insertion into the viable layers of the skin of a microneedle comprising a layer forming a tip and comprising a biodegradable sustained release polymer and one or more active ingredients . The microneedle further comprises a second layer on top of the first layer, the second layer comprising a polymer which dissolves shortly after insertion of the microneedle, thus permitting removal of a substrate on which the microneedle is attached. The microneedle composition exhibits desired physical and chemical stability and a desired rate of diffusion of the active ingredient from the polymer in which it is dispersed as well as a desired rate of degradation of the polymer to ensure release of the active ingredient over a prolonged period of time allowing less frequent dosing than the one or more times daily required when the composition is an ointment or cream.

Accordingly, in one aspect the present invention relates to a microneedle patch composition comprising one or more microneedles each comprising (a) a tapered tip portion containing a therapeutically active ingredient dispersed in a matrix of a biodegradable polymer capable of providing sustained release of the therapeutically active ingredient over a period of at least two days after insertion of the microneedle or microneedles into the skin, wherein the biodegradable polymer is polylactic acid or a derivative thereof such as an ester-terminated polylactide, polyglycolic acid or a derivative thereof such as an ester-terminated polyglycolide, or polylactic co-glycolic acid or a derivative thereof such as an ester-terminated polylactide co-glycolide and (b) a fast dissolving microneedle backing layer portion containing a water-soluble polymer overlayering the tip portion, said microneedle or microneedles being attached to and extending from an adhesive surface of a removable substrate, wherein the therapeutically active ingredient is released from the microneedles over a period of 2-14 days after insertion of the microneedle or microneedles into the skin.

### BRIEF DESCRIPTION OF THE DRAWINGS

Fig. 1a is a graphic representation of a microneedle patch of the invention before insertion into the skin. The portion of the microneedle shown in dark grey represents the fast dissolving backing layer comprising for instance polyvinylpyrrolidone, and the portion of the microneedle shown as cross-hatched represents the tip comprising the biodegradable polymer mixed with active ingredient(s) (shown as pale grey ovals).
Fig. 1b is a graphic representation of the microneedle patch shown in Fig. 1a after insertion into the skin and after the fast dissolving backing layer has dissolved. The tip portion of the microneedles is present in the viable layer(s) of the skin.
Fig. 1c is a graphic representation of the microneedle patch shown in Fig. 1b showing release of active ingredient(s) into the skin.
Fig. 2a is a schematic representation of a method of preparing a microneedle patch composition of the invention ("DMN" is an abbreviation of dissolvable microneedle).
Fig. 2b is a schematic representation of an alternative method of preparing a microneedle patch composition of the invention ("DMN" is an abbreviation of dissolvable microneedle).
Fig. 3 is a graph showing the peak area ratio of the degradation product MC 1046 to total concentration of calcipotriol in microneedles comprising ester-terminated polylactide co-glycolide (PLGA-E) in the tip portion and polyvinylpyrrolidone (PVP) in the backing layer in the presence or absence of the antioxidant butylhydroxytoluene (BHT) in samples taken after drying the microneedles for 5 hours at 65°C±2°C in a drying oven.
Fig. 4 is a graph showing the skin concentration (µM), 24 and 48 hours post application, of betamethasone-17,21-dipropionate (BDP) and betamethasone-17-propionate (B-17-P) in human skin explants treated with a microneedle patch composition of the invention compared to human skin explants treated with Daivobet® gel. B-17-P is predominantly formed in biological matrices and is thus considered a surrogate marker of BDP in skin.
Fig. 5a is a graph showing the mRNA levels, 24 and 48 hours post application, of CYP24A1 (a biomarker of calcipotriol exposure) in human skin explants treated with a microneedle patch composition of the invention compared to human skin explants treated with Daivobet® gel.
Fig. 5b is a graph showing the mRNA levels, 24 and 48 hours post application, of CD14 (a biomarker of calcipotriol exposure) in human skin explants treated with a microneedle patch composition of the invention compared to human skin explants treated with Daivobet® gel.
Fig. 6a is a graph showing the mRNA levels, 24 hours and 4 days post application, of CYP24A1 in human skin explants treated with a microneedle patch composition of the invention compared to human skin explants treated with Daivobet® gel.
Fig. 6b is a graph showing the skin concentration (µM), 24 hours and 4 days post application, of BDP and B-17-P in human skin explants treated with a microneedle patch composition of the invention compared to human skin explants treated with Daivobet® gel.
Figs. 7a-7e show a series of reflectance confocal microscopy images of one microneedle after application of a 5x5 microneedle patch to human ex vivo skin and removal of the substrate (medical tape) after 45 minutes, taken by means of a Vivascope 1500 multilaser system at a wavelength of 785 nm.

### DETAILED DESCRIPTION OF THE INVENTION

### Definitions

In the present context, the term "calcipotriol" is intended to indicate a vitamin D analogue of the formula

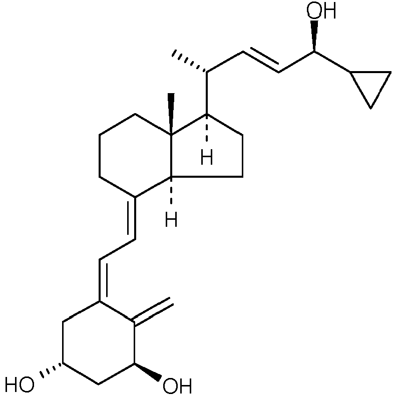

Calcipotriol has been found to exist in two crystalline forms, an anhydrate and a monohydrate. Calcipotriol monohydrate and its preparation are disclosed in WO 94/15912. The term "calcipotriol" is intended to cover any form of the compound, including crystalline, amorphous and dissolved forms.

The term "MC1046" is intended to indicate a compound of the formula

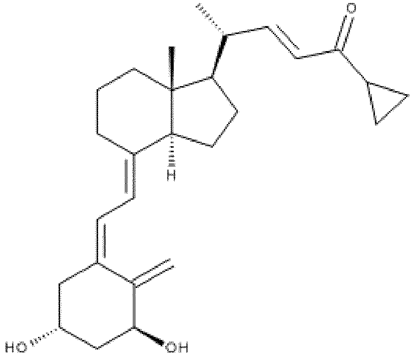

MC1046 is formed as a degradation product of calcipotriol under oxidative conditions.

The term "betamethasone ester" is intended to indicate a carboxylic acid ester of a compound of the formula

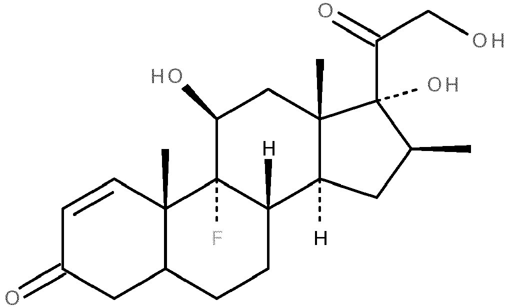

Examples of betamethasone esters that may be included in the present composition are betamethasone-17-valerate or betamethasone-17,21-dipropionate. A preferred betamethasone ester for the present purpose is betamethasone-17,21-dipropionate (referred to in the following as betamethasone dipropionate).

The term "dispersed" is intended to indicate that the active ingredient is either molecularly dispersed or present as a solid dispersion in the polymer matrix. Results from Raman spectroscopy of microneedle compositions of the invention suggest that both betamethasone dipropionate and calcipotriol are present as glass solutions molecularly dispersed in the polymer matrix.

The term "biodegradable" is intended to indicate that the polymer swells after insertion into the skin and subsequently degrades due to the hydrolysis of ester linkages in the presence of water.

The term "sustained release" is intended to indicate that the diffusion of active ingredient from the biodegradable polymer and/or the release of the active ingredient as a result of swelling, dissolution and/or degradation of the biodegradable polymer takes place over a prolonged period of time, such as at least two days, to enable delivery of a therapeutically effective dose of an active ingredient over the entire period, thus permitting less frequent dosing.

The term "fast dissolving" is intended to indicate that the backing layer portion of the microneedle dissolves within a period not exceeding 120 minutes, preferably not exceeding 60 minutes and which may be as short as about 15 minutes after insertion into the skin.

The term "chemical stability" or "chemically stable" is intended to indicate that no more than 10%, preferably no more than 5%, of either active ingredient degrades over the shelf-life of the product, which may be at least 1 year, but preferably at least 1.5 year or more preferably at least 2 years. An approximation of chemical stability at 5°C is obtained by subjecting the composition to accelerated stability studies at 25°C. If less than about 6% of the substance has degraded after 3 months at 25°C, this is usually taken to correspond to a shelf-life of 2 years at 5°C. More specifically, "chemical stability" of calcipotriol is intended to mean that the calcipotriol does not degrade significantly over time to 24-epi calcipotriol, MC1046 or other degradation products of calcipotriol in the finished pharmaceutical product.

The term "physical stability" or "physically stable" is intended to mean that the composition retains its macroscopic and microscopic appearance and physical properties over the shelf-life of the product. For instance, the composition is considered to be physically stable when the microneedles retain their shape and sharpness over time along with their mechanical strength as determined by the force required to provide sudden discontinuities (such as fractures) in the microneedle characteristic of sudden structural failure.

The term "ester-terminated" is intended to mean an alkyl ester of polylactic, polyglycolic or polylactic co-glycolic acid. The alkyl is preferably a C₁₋₂₀ alkyl, such as methyl, ethyl, propyl, butyl, pentyl, hexyl, heptyl, octyl, nonyl, decyl, undecyl, dodecyl, etc.

### A method of preparing the microneedle patch composition of the invention

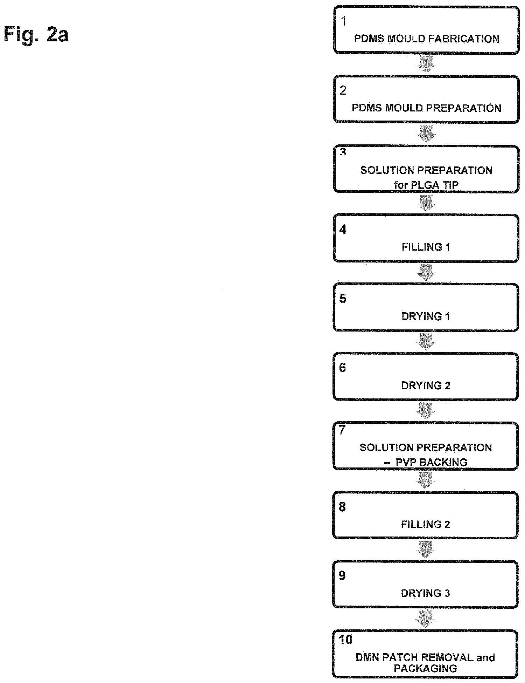

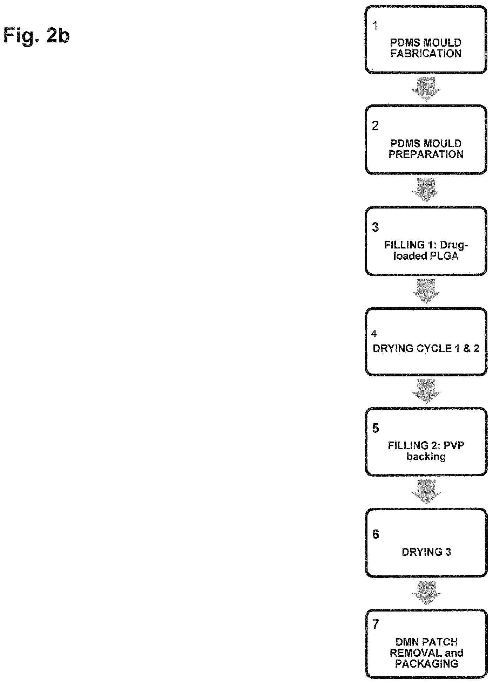

A method of preparing a microneedle patch composition of the present invention is shown schematically in Fig. 2. To make a mould for casting the microneedles, a template is initially prepared from a suitable material such as silicon or a metal such as steel or titanium or a polymer such as polycarbonate or polymethacrylic acid. The template comprise a plurality of microneedles which have a size and shape corresponding to the desired shape of the microneedles in the patch composition, i.e. typically either conical or pyramidal with a tapering tip. The mould may then be made by casting a liquid polymer material such as polydimethylsiloxane over the microneedle template. When the material is dried and cured the mould comprises microdepressions that retain the negative shape of the microneedles on which it is cast.

The length and number of microdepressions in the mould determine the length and number of the microneedles in the final patch. The number of microneedles per unit area may vary widely, typically between 2 and 100 microneedles per cm², but is more often in the range of 5-75 microneedles, more usually 10-50 microneedles, preferably 15-30 microneedles such as 20-25 microneedles per cm². Similarly, the length of the microneedles may vary, but they should be of sufficient length to penetrate the stratum corneum and not so long as to penetrate into the innervated part of the skin below the dermis as penetration to this depth may cause a painful sensation when the microneedle patch is applied on the skin. The microneedles may therefore have a length of 50-1000 µm, e.g. 100-800 µm, 300-700 µm, 400-600 µm or about 500 µm. Such a length of the microneedles generally ensures that the drug-loaded tip portion will lodge in the viable layers of the skin, i.e. the dermis and epidermis where the active ingredients exert their effect. As indicated above, the shape of the microneedles is also determined by the shape of the microdepressions in the mould and may be conical or pyramidal in shape and with a sharp tip. Furthermore, when the shape of the microneedle is pyramidal, it comprises a number, typically 4-8, of longitudinally extending ridges to facilitate the insertion of the microneedles into the skin. The aspect ratio of the microneedle (the length to width at the base of the microneedle) may vary according to the method used to produce the mould. When the mould template is prepared by wet etching (e.g. as described in US 2007/0134829), the aspect ratio is typically 3:2, i.e. the length is 1.5 times greater than the width at the base.

Microneedle patches according to the invention may then be made by filling the microdepressions in the mould with an appropriate solvent, e.g. dimethylformamide, and applying a solution of the active ingredients and the biodegradable polymer in the same solvent on top of the microdepressions to allow mixing of the two liquids. The solvent is then removed, e.g. by drying in a desiccator under vacuum and/or in a drying oven at an appropriate temperature. A second solution of the water-soluble polymer in an appropriate solvent is then applied on top of the partially filled microdepressions followed by drying, e.g. in a desiccator under vacuum or in a drying oven at an appropriate temperature. In a currently preferred embodiment, the solution of the water-soluble polymer is applied in such a manner that the backing layer portion when dried overlayers the base of the tip portion in such a way that each microneedle is separated from the other microneedles on the patch and forms a discrete entity when the substrate is removed upon application of the patch on the skin.

Depending on the dose of the active ingredients to be delivered from each patch, the drug-loaded tip portion may constitute 5-95% of the total volume of the microneedle.

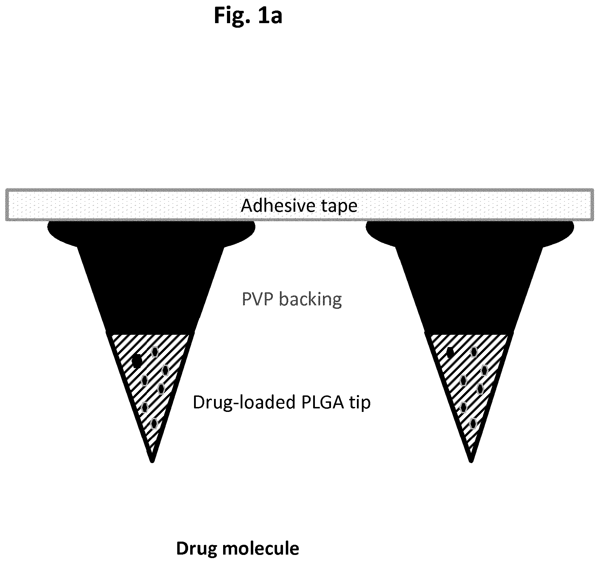

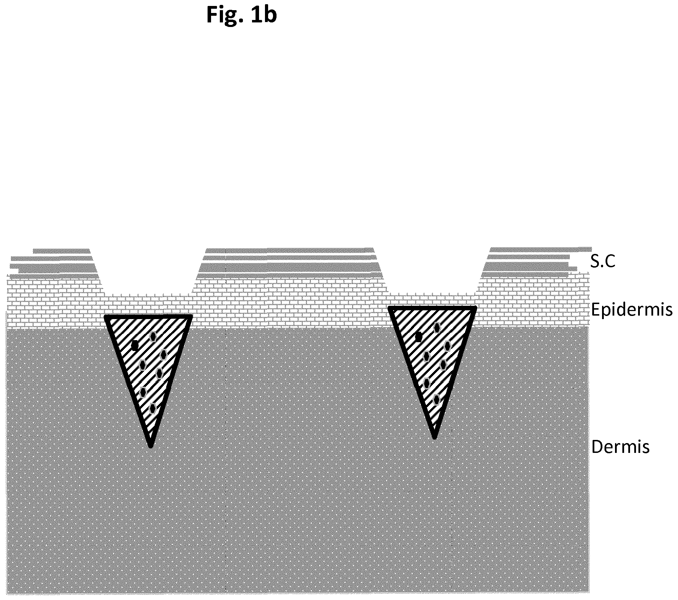

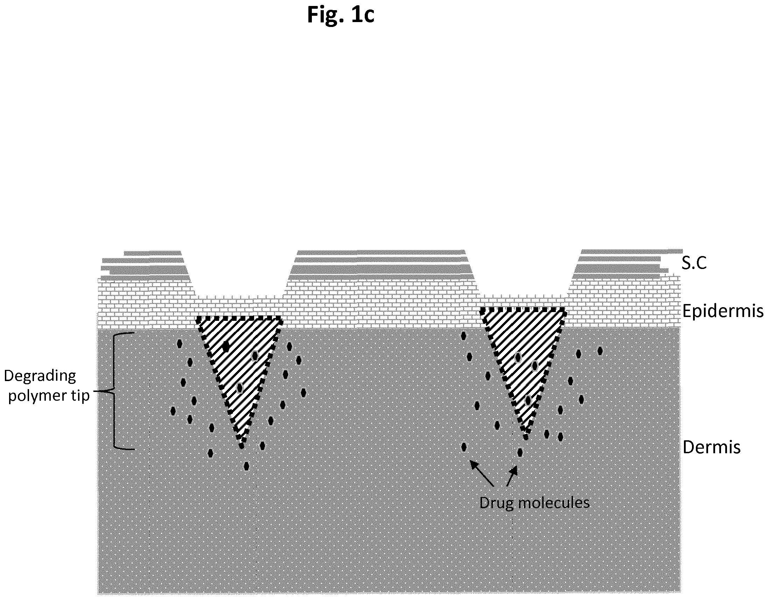

The dried microneedles may then be removed from the mould by applying adhesive tape on top of the mould and applying pressure to ensure good contact between the tape and the base of the microneedles followed by pulling the microneedles out of the mould. The tape should preferably be adhesive medical tape as this has been found to provide good adhesion to the base of the microneedles so that subtantially all microneedles are removed from the mould when the tape is pulled. The composition of the resulting microneedles is shown graphically in Fig. 1a. The mould may either be cast to match the desired size of each patch or may be made in a larger size, and individual patches of an appropriate size may be prepared by cutting the tape into pieces of a desired shape and size. The latter option may be as advantage when treating psoriasis as psoriasis plaques are often of different sizes and shapes.

The dried microneedle patches may be stored in a sealed airtight vial or blister pack, optionally together with an appropirate dessicant, to prevent absorption of water vapour during storage.

Further details of microneedle preparation and alternative embodiments are disclosed in WO 2012/153266.

### Embodiments

In the course of research leading to the present invention, a large number of different biodegradable polymers were tested for their suitability to form a matrix from which the microneedles could be made. The majority of the tested polymers were found to be unsuitable for the preparation of microneedles of the present invention either because the microneedles prepared from them did not retain their shape, in particular their sharp tip, i.e. they were not physically stable, or because the therapeutically active ingredient(s) were released unacceptably quickly from the polymer due to fast dissolution thereof, or because the therapeutically active ingredient(s) were found to be chemically unstable therein. Thus, microneedles made from polyvinylpyrrolidone and poly(meth)acrylates or mixtures of these polymers resulted in an unacceptably fast release of the active ingredient(s). Microneedles made from polyvinylpyrrolidone alone tended to soften or melt when removed from the primary packaging.

In the end, these various problems were solved by developing a microneedle composition comprising a drug-loaded tip portion containing a polylactic acid or a derivative thereof such as an ester-terminated polylactide, polyglycolic acid or a derivative thereof such as an ester-terminated polyglycolide or polylactic co-glycolic acid (PLGA) or a derivative thereof such as an ester-terminated polylactide co-glycolide polymer. Satisfactory results were also obtained when a water-soluble polymer such as polyvinylpyrrolidone was used as the backing layer. It has been found that when a polylactic acid, polyglycolic acid or polylactic co-glycolic acid polymer (or an ester-terminated derivative of these polymers) was employed it was possible to obtain a composition from the active ingredient(s) are released over a prolonged period of time such as at least two days. When polyvinylpyrrolidone was used as the backing layer, the resulting microneedles were found to be physically stable, with a hard, sharp tip.

In a particularly favoured embodiment, the biodegradable polymer is PLGA which may optionally be ester-terminated. The PLGA may favourably have a molecular weight of >5000, such as a molecular weight of 7000-17000, 24000-38000, 38000-54000, 54000-69000 or 76000-116000, resulting in a viscosity that permits the formulation to be dipensed into the mould and on drying provides a satisfactory physical stability, in particular a sharp tip. The ratio of lactic acid to glycolic acid may preferably vary between 85:15 and 50:50, such as 85:15, 82:18, 75:25, 65:35 or 50:50. A currently preferred ratio of lactide to glycolide is 50:50.

In some cases it may be preferred to add an antioxidant to the biodegradable polymer matrix, e.g. butylhydroxytoluene, butylhydroxyanisole or α-tocopherol, or a mixture thereof, so as to reduce the formation of degradation products of the active ingredient under oxidative conditions. The antioxidant may suitably be present in a concentration in the range of 0.01-3% w/w, preferably 0.03-2% w/w such as 0.05-1% w/w of the dry tip portion. A currently preferred antioxidant is butylhydroxytoluene, which may be added in a concentration in the range of 0.03-2% by weight, e.g. 0.05% by weight, of the dry tip portion.

The water-soluble polymer included in the backing layer may be any polymer that dissolves quickly in the skin after the composition has been applied and which is compatible with the other components. The water-soluble polymer may for instance be selected from the group consisting of polyvinylpyrrolidone, a sugar such as sucrose or trehalose, dextran, carboxymethylcellulose or sodium alginate. A currently preferred water-soluble polymer is polyvinylpyrrolidone. While generally the use of polyvinylpyrrolidone confers favourable properties to the backing layer portion in terms of physical stability of the microneedles, it may be somewhat brittle and its properties may be improved by including a plasticizer, e.g. glycerol, polyethylene glycol, dibutyl sebacate, diethyl phthalate, triethyl glycerin or triethyl citrate, to reduce the brittleness. The concentration of the plasticizer in the backing layer portion may suitably be in the range of 0.5-6% by weight of the dry backing layer. A currently favoured plasticizer to include in the backing layer portion of the microneedles is glycerol, which may suitably be present in a concentration of about 2% by weight of the dry backing layer. It should be noted that the residual solvent remaining in the backing layer after the composition has been dried may also act as a plasticizer. An example of such a solvent is ethanol which may be present as a residue in the composition after drying.

In a specific embodiment, the present composition may comprise an active ingredient in the backing layer of the water-soluble polymer. This will provide immediate (i.e. within 2 hours or preferably 1 hour) release of a portion of the active ingredient(s) administered to the patient. The active ingredient included in the backing layer may be the same or different from the active ingredient included in the tip portion of the microneedle.

The active ingredient(s) included in the present composition may be active ingredient(s) that are suitable for the treatment of skin conditions and where less frequent dosing (less than once a day) is perceived as advantageous by patients. The active ingredient may suitably be selected from the group consisting of a vitamin D analogue, a glucocorticoid receptor modulator, ingenol or an ingenol derivative, a calcineurin inhibitor, a JAK inhibitor, a PDE4 inhibitor, a non-steroidal anti-inflammatory agent, an antibiotic, an antifungal agent or a local anesthetic, or mixtures thereof.

Examples of vitamin D analogues are calcipotriol, calcitriol, maxacalcitol or tacalcitol.

Examples of glucocorticoid receptor modulators are corticosteroids such as amcinonide, betamethasone, budenoside, clobetasol, clobetasone, cortisone, desonide, desoxycortisone, desoximethasone, dexamethasone, diflucortolon, diflorasone, flucortisone, flumethasone, flunisolide, fluocinonide, fluocinolon, fluorometholone, fluprednisolone, flurandrenolide, fluticasone, halcinonide, halobetasol, hydrocortisone, meprednisone, methylprednisone, mometasone, paramethasone, prednicarbate, prednisone, prednisolone and triamcinolone or a pharmaceutically acceptable ester or acetonide thereof. The corticosteroid may preferably be selected from betamethasone, budenoside, clobetasol, clobetasone, desoximethasone, diflucortolon, diflorasone, fluocinonide, fluocinolon, halcinonide, halobetasol, hydrocortisone, mometasone prednicarbate, or triamcinolone or a pharmaceutically acceptable ester thereof. The corticosteroid ester may for instance be betamethasone acetate, betamethasone dipropionate, betamethasone valerate, clobetasol propionate, dexamethasone acetate, flumethasone pivalate, fluticasone propionate, hydrocortisone acetate, hydrocortisone butyrate or mometasone furoate. The acetonide may be selected from fluocinolone acetonide or triamcinolone acetonide.

An example of an ingenol derivative is ingenol mebutate.

Examples of calcineurin inhibitors are tacrolimus or pimecrolimus.

An example of a JAK inhibitor is tofacitinib.

Examples of PDE4 inhibitors are apremilast, roflumilast or cilomilast.

Examples of non-steroidal anti-inflammatory agents are ibuprofen, diclofenac, naproxen, indomethacin, dexibuprofen, ketoprofen, flurbiprofen, piroxicam, tenoxicam, lornoxicam or nabumeton.

Examples of antibiotics are fusidic acid or mupirocin.

Examples of local anesthetics are lidocain, bupivacain, mepivacain or ropivacain.

Examples of antifungal agents are ketoconazole, terbinafine, miconazole, clotrimazole, ciclopirox, bifonazole, nystatin, econazole or amorolfine.

The therapeutically active ingredient may also be selected from antiproliferative agents such as methotrexate or immunosuppressants such as cyclosporin.

In another aspect, the present invention relates to a method for treating a skin condition comprising
(a) applying a patch composition comprising one or more microneedles as described herein on a surface area of the skin of a patient in need of treatment,
(b) exerting sufficient force on the patch composition to permit the microneedles to penetrate through the stratum corneum and into the viable layers of the skin, and (c) removing the adhesive substrate from the patch composition.

To prevent the microneedles from breaking on insertion into the skin, the mechanical strength of the microneedles should be such that the force required to fracture the microneedle is significantly greater than the force required to insert the microneedle into the skin. Generally, the force required to insert a microneedle patch into the skin and have it penetrate past the stratum corneum is in the range of 0.4-8N, for instance 2-7N, such as 5N, per patch containing 25 microneedles per cm². The failure force of the microneedle can be assessed as either a fracture force or the force required to compress the microneedle by a defined length. These forces can be can be determined using a texture analyser (e.g. a TA.XT Plus Texture Analyzer, Stable Micro Systems, Surrey, UK) or using a computer-controlled force-displacement station (Model 5565, Instron, Buckinghamshire, UK).

As indicated above, the backing layer comprising the water-soluble polymer starts dissolving upon insertion of the microneedles into the skin. This allows removal of the substrate within about 120 minutes, preferably within 60 minutes or 45 minutes or even as little as about 15 minutes, of application of the patch on skin. In general, it is preferred that at least 90% of the microneedles detach from the adhesive surface upon removal of the substrate within this timeframe to avoid that a substantial number of the microneedles are pulled out again when the substrate is peeled off.

The invention has been found able to provide delivery of the therapeutically active ingredients over a prolonged period of time. Thus, the therapeutically active ingredients may be released from the microneedles over a period of 2-21 days, preferably 2-14 days such as 2-7 days or 4-7 days. As shown in Example 2 below, increased mRNA levels of the biomarker CYP24A1 are observed 4 days after application of a microneedle patch containing calcipotriol indicating that calcipotriol is released from the microneedles for at least 4 days.

In the present method, step (b) may be carried out by applying pressure with a finger or by impact insertion, e.g. by using an applicator device, the latter being preferred as it increases insertion reproducibility (cf. van der Maaden et al., AAPS Journal 16(4), July 2014, pp. 681-684. Examples of applicator devices are disclosed in US 2002/0123675 or WO 2008/091602.

Skin conditions to be treated using the microneedle patch composition of the invention may be selected from psoriasis, e.g. plaque psoriasis, inverse psoriasis, nail psoriasis or spot psoriasis, pustulosis palmoplantaris, actinic keratosis, squamous cell carcinoma, basal cell carcinoma, contact dermatitis, atopic dermatitis, eczema, hand eczema, warts, genital warts, alopecia, acne, rosacea or skin infections.

Psoriasis is a chronic inflammatory skin disease that manifests as erythematous, dry, scaling plaques resulting from hyperkeratosis. The plaques are most often found on the elbows, knees and scalp, though more extensive lesions may appear on other parts of the body, notably the lumbosacral region. A common treatment of mild to moderate psoriasis involves topical application of a composition containing a corticosteroid as the active ingredient. While efficacious, application of corticosteroids has the disadvantage of a number of adverse effects such as skin atrophy, striae, acneiform eruptions, perioral dermatitis, overgrowth of skin fungus and bacteria, hypopigmentation of pigmented skin and rosacea.

Combination products for the treatment of psoriasis have been marketed by LEO Pharma for a number of years under the trade names Daivobet® ointment and Daivobet® gel. The product comprises calcipotriol and betamethasone dipropionate as the active ingredients formulated in an ointment or gel vehicle comprising polyoxypropylene stearyl ether as a solvent. While the efficacy of the combination products is significantly superior to that of either active ingredient on its own, the products need to be applied once daily, and many patients, in particular those with extensive psoriatic lesions, would favour a greater ease of application such as less frequent application. It is considered desirable to further improve the biological efficacy of the combination of the two active ingredients by providing a formulation vehicle from which delivery of the active ingredients into the skin is prolonged compared to the commercial product.

Thus, in a currently favoured embodiment, the present invention relates to a microneedle patch composition comprising one or more microneedles each comprising
(a) a tapered tip portion containing one or more therapeutically active ingredients selected from the group consisting of calcipotriol and betamethasone esters dispersed in a matrix of a biodegradable polymer selected from the group consisting of ester-terminated polylactide, ester-terminated polyglycolide and ester-terminated polylactide co-glycolide, and
(b) a fast dissolving microneedle backing layer portion containing a water-soluble polymer overlayering the tip portion,
said microneedle or microneedles being attached to and extending from an adhesive surface of a removable substrate.

In this embodiment, the betamethasone ester may be betamethasone dipropionate or betamethasone valerate. The prolonged delivery is expected to be sustained with a dose of calcipotriol of 0.08-30 µg of calcipotriol per cm² of patch and a dose of betamethasone ester of 1-60 µg of betamethasone ester per cm² of patch. The betamethasone ester is preferably betamethasone dipropionate.

During development of this embodiment it was found that calcipotriol was not chemically stable in a matrix of polylactic acid, polyglycolic acid or polylactic co-glycolic acid, probably due to the presence of acidic residues or impurities therein, while calcipotriol was chemically stable when ester-terminated polylactide, polyglycolide or polylactide co-glycolide were used as the biodegradable polymer.

In this embodiment, the biodegradable polymer is preferably an ester-terminated polylactide co-glycolide. The ester-terminated polylactide co-glycolide may favourably have a molecular weight of >5000, such as a molecular weight of 7000-17000, 24000-38000, 38000-54000, 54000-69000 or 76000-116000, resulting in a viscosity that permts the formulation to be dispensed into the mould and on drying provides a satisfactory physical stability, in particular a sharp tip. The ratio of lactide to glycolide may preferably vary between 85:15 and 50:50, such as 85:15, 82:18, 75:25, 65:35 or 50:50. A currently preferred ratio of lactide to glycolide is 50:50.

In this embodiment, it may be preferred to add an antioxidant to the biodegradable polymer matrix, e.g. butylhydroxytoluene, butylhydroxyanisole or α-tocopherol, or a mixture thereof, so as to reduce the formation of MC 1046. The antioxidant may suitably be present in the concentration of the antioxidant is in the range of 0.03-3% w/w, preferably 0.05-2% w/w such as 0.05-1% w/w of the dry tip portion. A currently preferred antioxidant is butylhydroxytoluene, which may be added in a concentration in the range of 0.05-2% by weight of the dry tip portion.

In this embodiment, the water-soluble polymer may for instance be selected from the group consisting of polyvinylpyrrolidone, a sugar such as sucrose or trehalose, dextran, carboxymethylcellulose and sodium alginate. A currently preferred water-soluble polymer is polyvinylpyrrolidone. The backing layer portion may additionally comprise a plasticizer, e.g. glycerol, polyethylene glycol, dibutyl sebacate, diethyl phthalate, triethyl glycerin or triethyl citrate, which may be included in a concentration in the range of 0.5-6% by weight of the dry backing layer. A currently favoured plasticizer to include in the backing layer portion of the microneedles is glycerol, which may suitably be present in a concentration of about 2% by weight of the dry backing layer.

In a specific embodiment, the present composition may comprise calcipotriol and/or a betamethasone ester dispersed in the backing layer of the water-soluble polymer.

In this embodiment, the removable substrate may suitable be composed of adhesive medical tape.

The invention is further described in the following examples which are not in any way intended to limit the scope of the invention as claimed.

### EXAMPLES

### Example 1

### Compositions of the invention

A microneedle mould was prepared by mixing about 45 g of polydimethylsiloxane (PDMS) elastomer base (Sylgard 184 silicone elastomer kit, part A) and about 4.5 g of curing agent (Sylgard 184 silicone elastomer kit, part B) by hand using a spatula and beaker until thoroughly mixed. The resulting mixture was placed in a desiccator under vacuum for about 20 minutes. The PDMS was poured over a microneedle template (patterned silicon wafer obtained from the Tyndall National Institute, Ireland, and prepared essentially as disclosed in US 2007/0134829) and cured in a drying oven at 100°C for about 60 minutes. After cooling, the mould was peeled off the microneedle template and cut into individual moulds of 1x1 cm (containing 5x5 microdepressions).

The PDMS moulds were placed in a glass beaker and cleaned with dimethylformamide (DMF) under vacuum for 30 minutes and an ultrasonic bath for further 30 minutes at room temperature. The cleaned moulds were placed in a glass beaker and the microdepressions were prefilled with DMF under vacuum.

A solution was prepared by dissolving 300 mg ester-terminated polylactide co-glycolide (lactide:glycolide 50:50, Mw 7000-17000, PLGA-E) in 1 ml DMF using a vortex mixer for about 10 minutes until the PLGA-E was completely dissolved. In some embodiments, 0.5 mg/ml butylhydroxytoluene (BHT) was added to the PLGA-E solution. 20 mg calcipotriol and 40 mg betamethasone dipropionate (BDP) were dissolved in 1 ml of the resulting solution using a vortex mixer for about 10 minutes until the active ingredients were completely dissolved. The drug-loaded PLGA-E solution was dispensed into the microdepressions of the PDMS moulds prefilled with DMF using a syringe pump and capillary tube dispenser and a flow rate of 0.5 µl/min. to a total volume of 0.15±0.03 µl per mould.

The moulds were dried for about 18 hours in a desiccator under vacuum and subsequently in a vacuum oven under 500 mbars for about 5 hours at 60±2°C.

A second solution was prepared by dissolving 400 mg of polyvinylpyrrolidone (PVP, Kollidon® 17 PF) in 1 ml of ethanol 96% using a vortex mixer for about 5 minutes. 10 mg of glycerol was added and the mixture was stirred for 2-3 minutes using the vortex mixer. The PVP solution was dispensed into the microdepressions of the PDMS moulds containing the dried drug-loaded PLGA-E solution using a syringe pump and capillary tube dispenser at a flow rate of 1.5 µl/min. so that the dispensed volume was 0.75±0.15 µl per mould.

The filled moulds were dried in a desiccator under vacuum for about 2 hours.

Medical adhesive tape (3M) was applied on the surface of the moulds using finger pressure and the microneedles were pulled out of the mould.

The patches were stored in hermetically sealed vials purged with nitrogen and closed with a rubber stopper, aluminium cap and crimper.

The dried microneedle patch has the following composition.

| Ingredient | µg/patch | % w/w | mg/g |
|---|---|---|---|
| Betamethasone dipropionate | 6 | 1.66 | 16.60 |
| Calcipotriol monohydrate | 3 | 0.83 | 8.30 |
| PLGA-E | 45 | 12.45 | 124.48 |
| PVP | 300 | 82.99 | 829.88 |
| Glycerol 99.5% | 7.5 | 2.07 | 20.75 |
| Total | 378.82 | 100 | 1000 |

Physical and chemical stability of the composition appears from the following table. It should be noted that storage of the microneedle patches at 40°C, which is the usual temperature for accelerated stability studies, was not feasible since PLGA-E is not physically stable at 40°C.

| Storage temperature/ time | Appearance | Calcipotriol % of start | 24-epi-calcipotriol % area | MC1046 % area | BDP % of start |
|---|---|---|---|---|---|
| Start | OK | 100.0 % | 0.8 % | 1.2 % | 100.0 % |
| 25°C/ 1 month | OK | 95.5 % | 1.0 % | 1.5 % | 98.3 % |
| 25°C/ 3 months | Not evaluated | 116.4 % | 0.7 % | 2.1 % | 118.6 % |
| 40°C/ 2 weeks | Not acceptable | 95.5 % | 0.9 % | Not evaluated | 101.7 % |

Microneedle patch compositions were prepared as described above with the exception that they contained 0%, 0.5% or 1% BHT by weight of the dry tip.

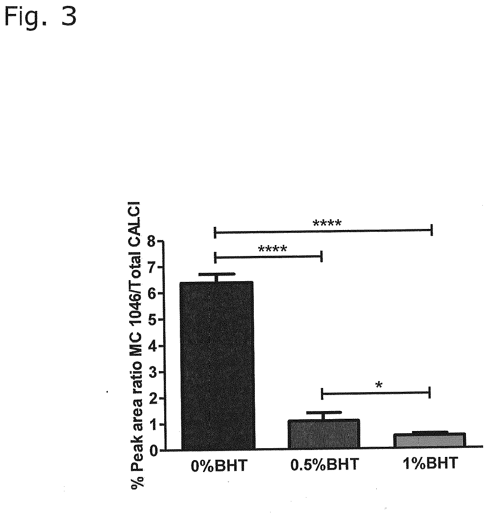

The results are shown in Fig. 3 which illustrates the percentage ratio of peak area of the degradation product MC 1046 to the total calcipotriol peak area for samples without BHT and samples with 0.5% w/w and 1% w/w BHT. The percentage peak area of MC 1046 relative to the total amount of calcipotriol was significantly reduced, indicating that the addition of BHT to the composition significantly reduced degradation of calcipotriol.

### Example 2

### Human explant skin exposure

Two experiments were performed to investigate exposure over time in human skin explants.

### Experiment 1:

Full-thickness human skin obtained from female donors undergoing abdominoplasty maximally 24 hours prior to the start of the experiment was used. 22 mm punch biopsies were placed in 24 mm Transwell® inserts and placed in 6 well plates with 1 ml EpiLife® tissue culture medium supplemented with 0.2 ng/mL human EGF, 0.2 % bovine pituitary extract (BPE), 5 µg/mL bovine insulin, 5 µg/mL bovine transferrin, 0.18 µg/mL hydrocortisone and gentamycin.

Compositions prepared as described in Example 1 containing 2 µg calcipotriol and 6 µg BDP per cm² microneedle patch and 10 µl Daivobet® gel per cm² and Daivobet® gel vehicle were applied topically in triplicate. The following treatment schedules were tested: One dose of Daivobet® gel at t=0h with skin sampling at 24h and 48h. Two doses of Daivobet® gel at t=0 and 24h respectively with skin sampling at 48h, one patch applied at t=0h with skin sampling at 24h and 48h leaving the backing tape on the skin for the full duration of the experiment, and one patch applied at t=0h with skin sampling at t=48h but removing the backing tape at 24h. The skin biopsies were maintained in *ex vivo* culture at 37°C with 5% CO₂ for 48 hours with a change of medium at 24 h. At the end of the experiment, a 14 mm biopsy encompassing the dosed area of each explant was punched out and subsequently divided in two for compound analysis (tapestripped 10 times) and biomarker analysis, respectively.

Compound analysis was performed by extracting the active compounds from the skin biopsy using an organic solvent and subsequently analysing the extract using LC/MS-MS.

Total RNA was extracted from cells using the mirVana (Life Technologies, Grand Island, NY, USA) according to the instructions provided. cDNA synthesis was performed with the High-Capacity cDNA Reverse Transcription Kit (Applied Biosystems, Foster City, CA, USA). 2.5 µL of cDNA (equivalent to 5 ng RNA) from each sample was amplified in a total volume of 10 µL by quantitative realtime PCR using Taqman Gene Expression Assays (CYP24A1 (Hs00167999_m1), CD14 (Hs02621496_s1), PPIA (Hs99999904_m1) GAPDH (Hs99999905_m1), TBP (Hs99999910_m1) and HMBS (Hs00609297_m1)) and PRISM7900HT sequence detection system (SDS 2.3) from Applied Biosystems. PPIA, GAPDH, TBP and HMBS were used for normalization.

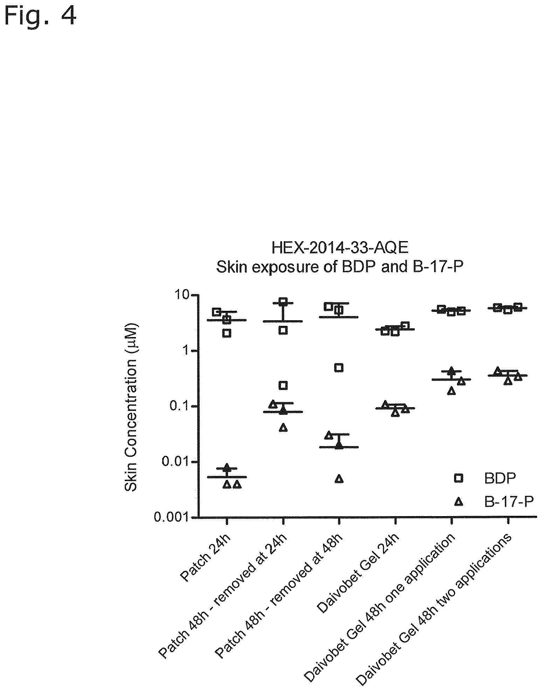

It appears from Fig. 4 that after application BDP could reside either inside microneedle patch compositions or in the stratum corneum of the skin after Daivobet® gel applications and thus be unavailable for pharmacological action. B-17-P is predominantly formed in biological matrices and is thus considered a surrogate marker of BDP available for pharmacological action in skin. It appears that the amount of B-17-P formed in the skin increases over time for both explants treated with Daivobet® gel and with microneedle patch compositions of the invention. The skin concentrations of B-17-P observed after application of Daivobet® gel are higher than what was observed after application of microneedle patch compositions of the invention, however the increase over time may indicate a prolonged release from the patches.

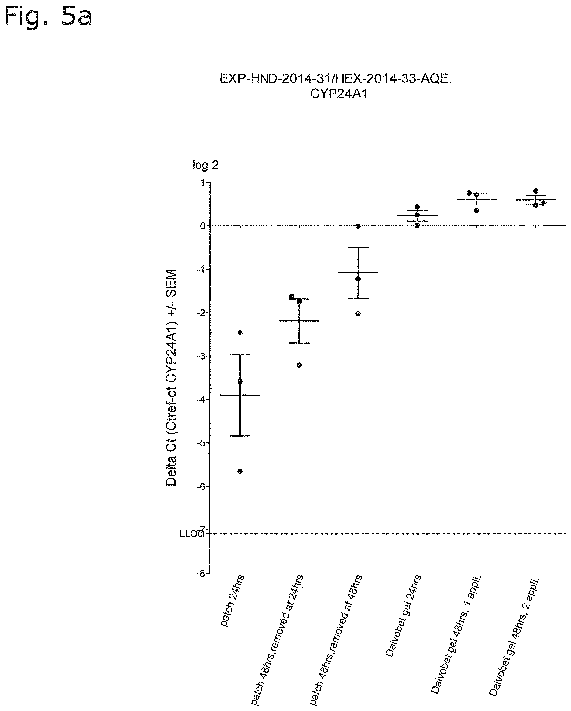

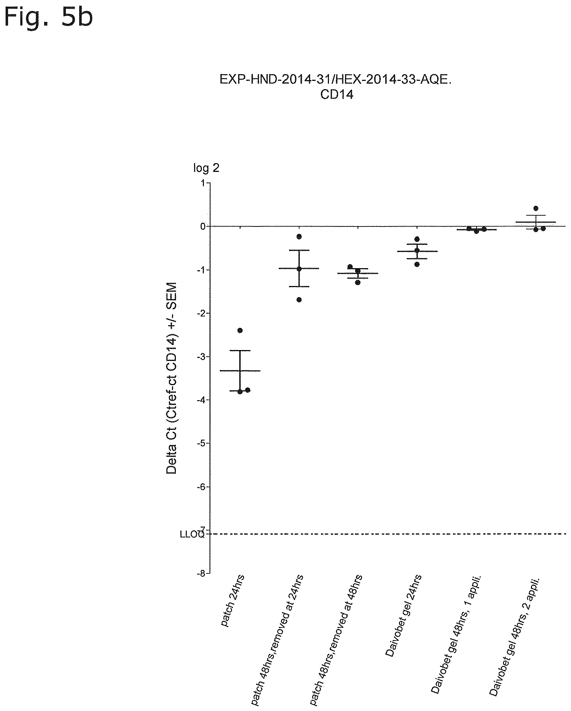

It appears from Figs. 5(a) and 5(b) that the PD biomarkers for calcipotriol, CYP24A1 and CD14, are induced over time by Daivobet® gel. The level of biomarker induction elicited by microneedle patches is lower, but increasing over time, indicating a slower onset but potentially a prolonged effect of calcipotriol than what is observed from Daivobet® gel.

### Experiment 2:

NativeSkin®Plus skin models with an available surface area of 2.5 cm² were acquired from Genoskin, France and cultured according to the manufacturer's specification. Compositions prepared as disclosed in Example 1 containing 0.5 µg calcipotriol and 6 µg BDP per cm² microneedle patch and 4.3 µl Daivobet® gel per cm² and Daivobet® gel vehicle were applied topically in triplicate. The following treatment schedules were tested: One daily dose of Daivobet® gel or placebo gel with skin sampling at 24h and 96h. One patch applied at t=0h with skin sampling at 24h and 96h leaving the backing tape on the skin for 24h, and two patches applied at t=0h and t=48h with skin sampling at t=96h. At the end of the experiment, two 4 mm biopsies were punched out and subsequently either analysed for compound (after being tapestripped 10 times) or the presence of biomarker.

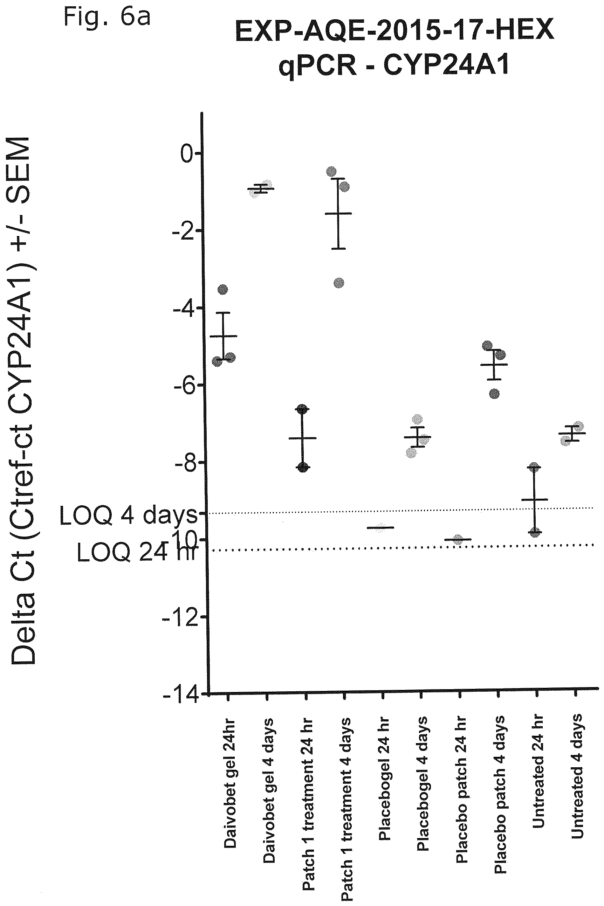

It appears from Fig. 6(a) that the biomarker for calcipotriol, CYP24A1, is induced over time by Daivobet® gel applied at time 0, day 1 and day 2 of the experiment and sampled on day 4. The level of biomarker induction elicited by the microneedle patch applied once is initially lower (at day 1), but increases over time, indicating a slower onset but potentially a protracted effect of calcipotriol over 4 days compared to what is observed from Daivobet® gel.

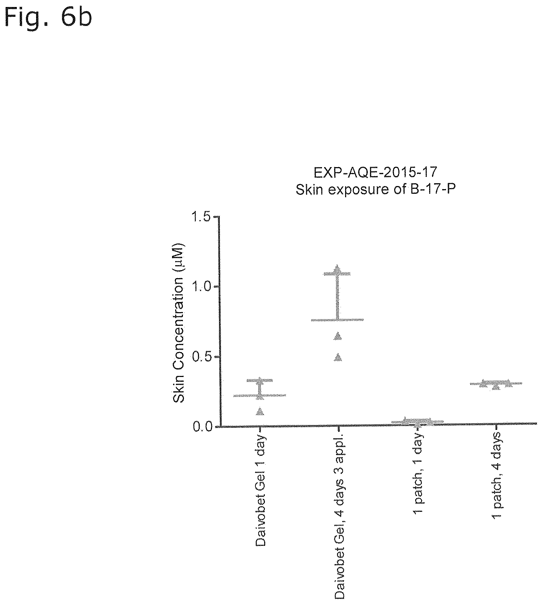

It appears from Fig. 6b that the amount of B-17-P formed in the skin increases over time for both explants treated with Daivobet® gel and with a microneedle patch composition of the invention. The skin concentrations of B-17-P observed after application of Daivobet® gel applied at time 0, day 1 and day 2 of the experiment are higher on day 4 than concentrations observed after application once of a microneedle patch composition of the invention, however the increase over time may indicate a prolonged release from the patches.

### Example 3

### Reflectance confocal microscopy of a microneedle composition in human explant skin

A microneedle patch as described in Example 1 was applied to fresh human ex vivo skin prepared as described in Example 2. 45 minutes after application the medical adhesive tape was removed and it was conformed that none of the microneedles was left on the tape before reflectance confocal microscopy (RCM) imaging was conducted using a Vivascope 1500 multilaser system in accordance with the procedure described in H. Skvara et al. Dermatol Pract Concept 2(1), 2012, pp.3-12, and Calzavara-Pinton et al., Photochemistry and Photobiology, 84, 2008, pp. 1421-30. In this technique laser light with a wavelength of 785 nm is passed through a beam splitter and an optical lens in contact with skin. In the skin, light is focused on a small tissue spot a few microns of diameter. Reflection (back scattering) occurs at the boundary between two structures having different indexes of refraction. Light reflected from the focal point propagates back toward the lens through a pinhole. Light reflected from above and below the point in focus is masked out by the pinhole so that the detector receives light only from the thin plane of the specimen that is in focus. By changing the depth at which the objective lens focuses in the vertical plane horizontal images can be generated at particular depths within the skin.

The scanned field of view was 500 x 500 µm. Depth measurements were done in steps of 3 µm with an axial resolution of < 5 µm. The limit of detection of the RCM is a depth of about 150 µm.

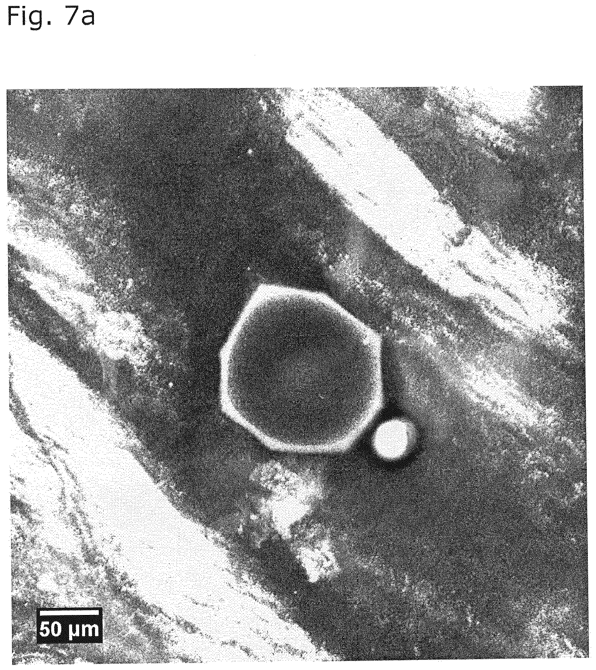

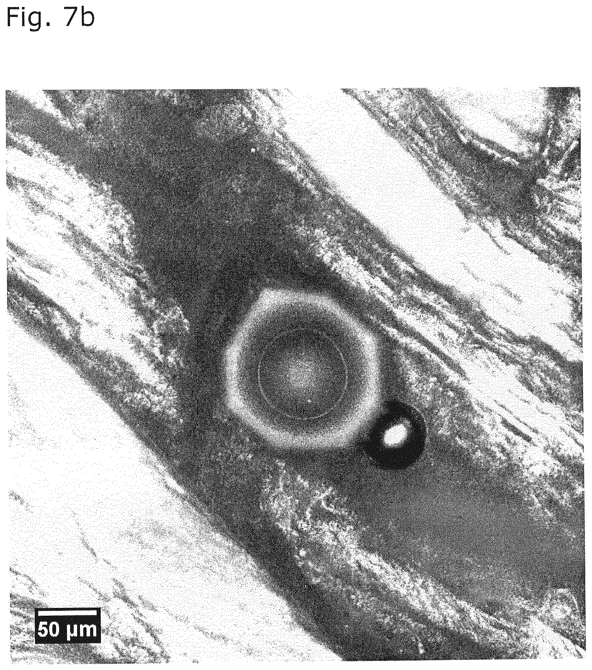

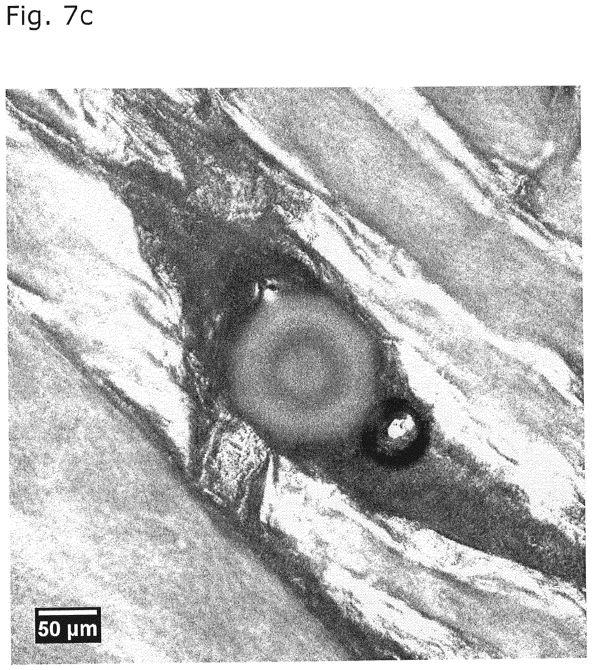

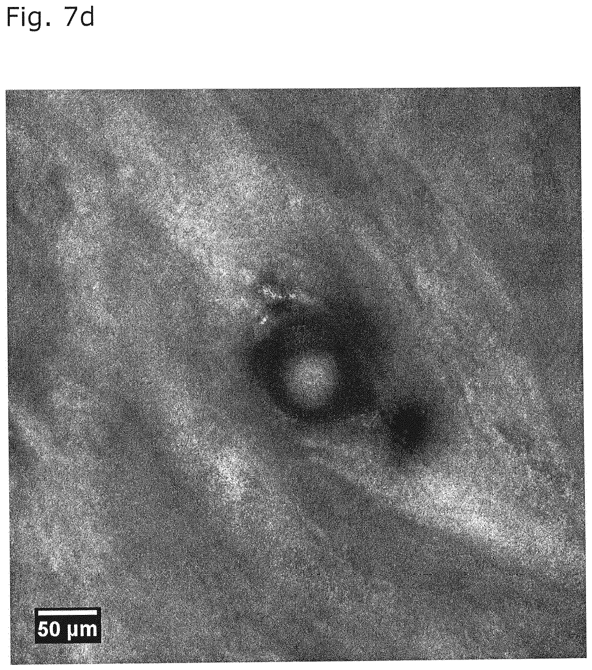

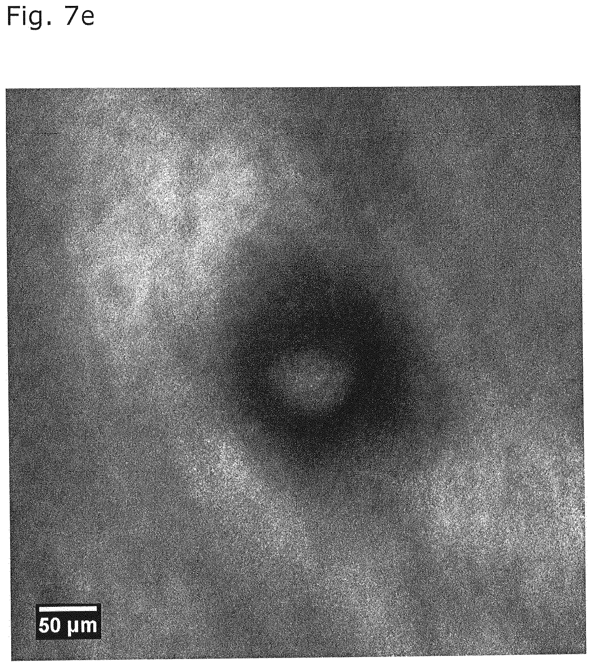

The results appear from Fig. 7, in which
Fig. 7a shows a microneedle penetrating the stratum corneum at a depth of 12 µm. The PVP backing layer has dissolved before the removal of the substrate and only a thin octagonal shell of the PLGA-E polymer reflects the light on this plane.
Fig. 7b shows a microneedle penetrating the stratum corneum at a depth of 27 µm. The PVP backing layer has dissolved before the removal of the substrate and only appears as a circle in the middle of a thin octagonal shell of the PLGA-E biodegradable polymer.
Fig. 7c shows a microneedle penetrating the stratum corneum at a depth of 45 µm. The PLGA-E polymer microneedle tip reflects the light on this plane as does a thin octagonal shell of the PLGA-E polymer.
Fig. 7d shows a microneedle penetrating the epidermis at a depth of 96 µm. Only the tip of the needle composed of the PLGA-E biodegradable polymer is visible.
Fig. 7e shows a microneedle penetrating the epidermis at a depth of 150 µm. Only the tip of the needle composed of the PLGA-E biodegradable polymer is visible at this depth.

## Claims

1. A microneedle patch composition comprising one or more microneedles each comprising
(a) a tapered tip portion containing a therapeutically active ingredient dispersed in a matrix of a biodegradable polymer capable of providing sustained release of the therapeutically active ingredient over a period of at least two days after insertion of the microneedle or microneedles into the skin, wherein the biodegradable polymer is polylactic acid or an ester-terminated polylactide, polyglycolic acid or an ester-terminated polyglycolide, or polylactic co-glycolic acid or an ester-terminated polylactide co-glycolide and
(b) a fast dissolving microneedle backing layer portion containing a water-soluble polymer overlayering the tip portion,
said microneedle or microneedles being attached to and extending from an adhesive surface of a removable substrate,
wherein the therapeutically active ingredient is released from the microneedles over a period of 2-14 days after insertion of the microneedle or microneedles into the skin.

2. A patch composition according to claim 1 comprising 2-100 microneedles per cm², e.g. 5-75 microneedles, 10-50 microneedles, 15-30 microneedles or 20-25 microneedles per cm².

3. A patch composition according to any one of claims 1-2, wherein the biodegradable polymer has a molecular weight of >5000, such as a molecular weight of 7000-17000, 24000-38000, 38000-54000, 54000-69000 or 76000-116000.

4. A patch composition according to claim 2 or3, wherein the ratio of lactide to glycolide is between 85:15 and 50:50, such as 85:15, 82:18, 75:25, 65:35 or 50:50.

5. A patch composition according to any one of claims 1-4, wherein the biodegradable polymer matrix further comprises an antioxidant, e.g. butylhydroxytoluene, butylhydroxyanisole or α-tocopherol, or a mixture thereof.

6. A patch composition according to any one of claims 1-5, wherein the water-soluble polymer is selected from the group consisting of polyvinylpyrrolidone, a sugar such as sucrose or trehalose, dextran, carboxymethylcellulose and sodium alginate.

7. A patch composition according to claim6, wherein the water-soluble polymer is polyvinylpyrrolidone.

8. A patch composition according to any one of claims 1-7, wherein the backing layer portion comprises a plasticizer, e.g. glycerol, polyethylene glycol, dibutyl sebacate, diethyl phthalate, triethyl glycerin or triethyl citrate.

9. A patch composition according to any one of claims 1-8, wherein the backing layer portion overlayers the base of the tip portion in such a manner that each microneedle is separated from the other microneedles on the patch and forms a discrete entity when the substrate is removed upon application of the patch on the skin.

10. A patch composition according to any one of claims 1-9, wherein the microneedles are either conical or pyramidal thus comprising a number of longitudinally extending ridges to facilitate the insertion of the microneedles into the skin.

11. A patch composition according to any one of claims 1-10, wherein the therapeutically active ingredient is selected from the group consisting of a vitamin D analogue, a glucocorticoid receptor modulator, ingenol or an ingenol derivative, a calcineurin inhibitor, a JAK inhibitor, a PDE4 inhibitor, a non-steroidal anti-inflammatory agent, an antibiotic, an antifungal agent or a local anesthetic, or mixtures thereof.

12. A patch composition according to any one of claims 1-11 comprising one or more microneedles each comprising
(a) a tapered tip portion containing one or more therapeutically active ingredients selected from the group consisting of calcipotriol and a betamethasone ester dispersed in a matrix of a biodegradable polymer selected from the group consisting of an ester-terminated polylactide, an ester-terminated polyglycolide and an ester-terminated polylactide co-glycolide, and
(b) a fast dissolving microneedle backing layer portion containing a water-soluble polymer overlayering the tip portion,
said microneedle or microneedles being attached to and extending from an adhesive surface of a removable substrate.

13. A patch composition according to claim 12, wherein the betamethasone ester is betamethasone dipropionate or betamethasone valerate, in particular betamethasone dipropionate.

14. A patch composition according to claim 12 or 13 comprising 0.08-30 µg of calcipotriol per cm².

15. A patch composition according to any one of claims 12-14 comprising 1-60 µg of betamethasone dipropionate per cm².

16. A patch composition according to any one of claims 12-15, wherein the biodegradable polymer is an ester-terminated polylactide co-glycolide.

17. A patch composition according to any one of claims 12-16, wherein the biodegradable polymer has a molecular weight of >5000, such as a molecular weight of 7000-17000, 24000-38000, 38000-54000, 54000-69000 or 76000-116000.

18. A patch composition according to claim 16 or 17, wherein the ratio of lactide to glycolide is between 85:15 and 50:50, such as 85:15, 82:18, 75:25, 65:35 or 50:50.

19. A patch composition according to any one of claims 12-18, wherein the backing layer comprises calcipotriol and/or a betamethasone ester dispersed in the matrix of the water-soluble polymer.

20. A patch composition according to any one of claims 12-19, wherein the water-soluble polymer is polyvinylpyrrolidone.

21. A microneedle patch composition for use in treating a skin condition, the composition comprising one or more microneedles each comprising
(a) a tapered tip portion containing a therapeutically active ingredient dispersed in a matrix of a biodegradable polymer capable of providing sustained release of the therapeutically active ingredient over a period of at least two days after insertion of the microneedle or microneedles into the skin, wherein the biodegradable polymer is polylactic acid or an ester-terminated polylactide, polyglycolic acid or an ester-terminated polyglycolide, or polylactic co-glycolic acid or an ester-terminated polylactide co-glycolide and
(b) a fast dissolving microneedle backing layer portion containing a water-soluble polymer overlayering the tip portion,
said microneedle or microneedles being attached to and extending from an adhesive surface of a removable substrate
wherein the therapeutically active ingredient is released from the microneedles over a period of 2-14 days after insertion of the microneedle or microneedles into the skin.

22. A patch composition for use according to claim 21, wherein the biodegradable polymer has a molecular weight of >5000, such as a molecular weight of 7000-17000, 24000-38000, 38000-54000, 54000-69000 or 76000-116000.

23. A patch composition for use according to claim 21 or 22, wherein the ratio of lactide to glycolide is between 85:15 and 50:50, such as 85:15, 82:18, 75:25, 65:35 or 50:50.

24. A patch composition for use according to any one of claims 21-23, wherein the water-soluble polymer is selected from the group consisting of polyvinylpyrrolidone, a sugar such as sucrose or trehalose, dextran, carboxymethylcellulose and sodium alginate.

25. A patch composition for use according to claim 24, wherein the water-soluble polymer is polyvinylpyrrolidone.

26. A patch composition for use according to any one of claims 21-25, wherein the therapeutically active ingredient is selected from the group consisting of a vitamin D analogue, a glucocorticoid receptor modulator, ingenol or an ingenol derivative, a calcineurin inhibitor, a JAK inhibitor, a PDE4 inhibitor, a non-steroidal anti-inflammatory agent, an antibiotic, an antifungal agent or a local anesthetic, or mixtures thereof

27. A patch composition for use according to any one of claims 21-26, wherein the skin condition is psoriasis, actinic keratosis, squamous cell carcinoma, basal cell carcinoma, contact dermatitis, atopic dermatitis, eczema, hand eczema, warts, genital warts, alopecia, acne, rosacea or skin infections.

## Patentansprüche

1. Mikronadel-Pflaster-Zusammensetzung, die eine oder mehrere Mikronadeln umfasst, die jeweils folgendes umfassen:
(a) einen konischen Spitzenteil, der einen therapeutisch wirksamen Inhaltsstoff enthält, der in einer Matrix eines biologisch abbaubaren Polymers verteilt ist, die eine retardierte Freisetzung des therapeutisch wirksamen Inhaltsstoffs über einen Zeitraum von mindestens zwei Tagen nach Einführung der Mikronadel oder der Mikronadeln in die Haut bereitstellt, wobei das biologisch abbaubare Polymer Polymilchsäure oder ein Ester-terminiertes Polylactid, Polyglykolsäure oder ein Ester-terminiertes Polyglykolid oder Polymilchsäure-Polyglykolsäure-Copolymer oder ein Ester-terminiertes Polymilchsäure-Polyglykolsäure-Copolymer ist; und
(b) einen sich schnell auflösenden Mikronadel-Rückseitenschichtteil, der ein wasserlösliches Polymer enthält, das den Spitzenteil überlagert,
wobei die Mikronadel oder die Mikronadeln an einer Klebefläche eines entfernbaren Substrats angebracht sind und sich von dort erstrecken,
wobei der therapeutisch wirksame Inhaltsstoff von den Mikronadeln nach der Einführung der Mikronadel oder der Mikronadeln in die Haut über einen Zeitraum von 2 bis 14 Tagen freigesetzt wird.

2. Pflaster-Zusammensetzung nach Anspruch 1, die 2-100 Mikronadeln pro cm² umfasst, wie zum Beispiel 5-75 Mikronadeln, 10-50 Mikronadeln, 15-30 Mikronadeln oder 20-25 Mikronadeln pro cm².

3. Pflaster-Zusammensetzung nach einem der Ansprüche 1 bis 2, wobei das biologisch abbaubare Polymer ein Molekulargewicht von >5.000 aufweist, wie etwa ein Molekulargewicht von 7.000-17.000, 24.000-38.000, 38.000-54.000, 54.000-69.000 oder 76.000-116.000.

4. Pflaster-Zusammensetzung nach Anspruch 2 oder 3, wobei das Verhältnis von Lactid zu Glykolid zwischen 85:15 und 50:50 liegt, wie etwa 85:15, 82:18, 75:25, 65:35 oder 50:50.

5. Pflaster-Zusammensetzung nach einem der Ansprüche 1 bis 4, wobei die biologisch abbaubare Polymermatrix ferner ein Antioxidationsmittel umfasst, zum Beispiel Butylhydroxytoluol, Butylhydroxyanisol oder α-Tocopherol oder ein Gemisch davon.

6. Pflaster-Zusammensetzung nach einem der Ansprüche 1 bis 5, wobei das wasserlösliche Polymer ausgewählt ist aus der Gruppe bestehend aus Polyvinylpyrrolidon, einem Zucker, wie etwa Saccharose oder Trehalose, Dextran, Carboxymethylcellulose und Natriumalginat.

7. Pflaster-Zusammensetzung nach Anspruch 6, wobei das wasserlösliche Polymer Polyvinylpyrrolidin ist.

8. Pflaster-Zusammensetzung nach einem der Ansprüche 1 bis 7, wobei der Rückseitenschichtteil einen Weichmacher umfasst, zum Beispiel Glycerol, Polyethylenglykol, Dibutylsebacat, Diethylphthalat, Triethylglycerin oder Triethylcitrat.

9. Pflaster-Zusammensetzung nach einem der Ansprüche 1 bis 8, wobei der Rückseitenschichtteil die Basis des Spitzenteils so überlagert, dass jede Mikronadel von den anderen Mikronadeln auf dem Pflaster getrennt ist und eine diskrete Einheit bildet, wenn das Substrat nach dem Auftragen des Pflasters auf die Haut entfernt wird.

10. Pflaster-Zusammensetzung nach einem der Ansprüche 1 bis 9, wobei die Mikronadeln entweder konisch oder pyramidenförmig sind, so dass sie eine Anzahl sich längs erstreckender Grate umfassen, um die Einführung der Mikronadeln in die Haut zu erleichtern.

11. Pflaster-Zusammensetzung nach einem der Ansprüche 1 bis 10, wobei der therapeutisch wirksame Inhaltsstoff ausgewählt ist aus der Gruppe bestehend aus einem Vitamin D-Analogon, einem Glukokortikoid-Rezeptor-Modulator, Ingenol oder einem Ingenolderivat, einem Calcineurinhemmer, einem JAK-Hemmer, einem PDE4-Hemmer, einer nicht steroidalen entzündungshemmenden Substanz, einem Antibiotikum, einem Antimykotikum oder einem Lokalanästhetikum oder Gemischen davon.

12. Pflaster-Zusammensetzung nach einem der Ansprüche 1 bis 11, die eine oder mehrere Mikronadeln umfasst, die jeweils folgendes umfassen:
(a) einen konischen Spitzenteil, der einen oder mehrere therapeutisch wirksame Inhaltsstoffe enthält, die ausgewählt sind aus der Gruppe bestehend aus Calcipotriol und einem Betamethasonester, verteilt in einer Matrix eines biologisch abbaubaren Polymers, das ausgewählt ist aus der Gruppe bestehend aus Ester-terminiertem Polylactid, Ester-terminiertem Polyglykolid oder Ester-terminiertem Polymilchsäure-Polyglykolsäure-Copolymer; und
(b) einen sich schnell auflösenden Mikronadel-Rückseitenschichtteil, der ein wasserlösliches Polymer enthält, das den Spitzenteil überlagert,
wobei die Mikronadel oder Mikronadeln an einer Klebefläche eines entfernbaren Substrats angebracht sind und sich von dort erstrecken.

13. Pflaster-Zusammensetzung nach Anspruch 12, wobei der Betamethasonester Betamethasondipropionat oder Betamethasonvalerat ist, im Besonderen Betamethasondipropionat.

14. Pflaster-Zusammensetzung nach Anspruch 12 oder 13, die 0,08-30 µg Calcipotriol pro cm² umfasst.

15. Pflaster-Zusammensetzung nach einem der Ansprüche 12 bis 14, die 1-60 µg Betamethasondipropionat pro cm² umfasst.

16. Pflaster-Zusammensetzung nach einem der Ansprüche 12 bis 15, wobei das biologisch abbaubare Polymer ein Ester-terminiertes Polymilchsäure-Polyglykolsäure-Copolymer ist.

17. Pflaster-Zusammensetzung nach einem der Ansprüche 12 bis 16, wobei das biologisch abbaubare Polymer ein Molekulargewicht von >5.000 aufweist, wie etwa ein Molekulargewicht von 7.000-17.000, 24.000-38.000, 38.000-54.000, 54.000-69.000 oder 76.000-116.000.

18. Pflaster-Zusammensetzung nach Anspruch 16 oder 17, wobei das Verhältnis von Lactid zu Glykolid zwischen 85:15 und 50:50 liegt, wie etwa 85:15, 82:18, 75:25, 65:35 oder 50:50.

19. Pflaster-Zusammensetzung nach einem der Ansprüche 12 bis 18, wobei die Rückseitenschicht Calcipotriol und/oder Betamethasonester verteilt in der Matrix des wasserlöslichen Polymers umfasst.

20. Pflaster-Zusammensetzung nach einem der Ansprüche 12 bis 19, wobei das wasserlösliche Polymer Polyvinylpyrrolidon ist.

21. Mikronadel-Pflaster-Zusammensetzung zur Verwendung in der Behandlung eines Hautzustands, wobei die Zusammensetzung eine oder mehrere Mikronadeln umfasst, die jeweils folgendes umfassen:
(a) einen konischen Spitzenteil, der einen therapeutisch wirksamen Inhaltsstoff enthält, der in einer Matrix eines biologisch abbaubaren Polymers verteilt ist, die eine retardierte Freisetzung des therapeutisch wirksamen Inhaltsstoffs über einen Zeitraum von mindestens zwei Tagen nach Einführung der Mikronadel oder der Mikronadeln in die Haut bereitstellt, wobei das biologisch abbaubare Polymer Polymilchsäure oder ein Ester-terminiertes Polylactid, Polyglykolsäure oder ein Ester-terminiertes Polyglykolid oder Polymilchsäure-Polyglykolsäure-Copolymer oder ein Ester-terminiertes Polymilchsäure-Polyglykolsäure-Copolymer ist; und
(b) einen sich schnell auflösenden Mikronadel-Rückseitenschichtteil, der ein wasserlösliches Polymer enthält, das den Spitzenteil überlagert,
wobei die Mikronadel oder die Mikronadeln an einer Klebefläche eines entfernbaren Substrats angebracht sind und sich von dort erstrecken,
wobei der therapeutisch wirksamen Inhaltsstoff von den Mikronadeln nach der Einführung der Mikronadel oder der Mikronadeln in die Haut über einen Zeitraum von 2 bis 14 Tagen freigesetzt wird.

22. Pflaster-Zusammensetzung zur Verwendung nach Anspruch 21, wobei das biologisch abbaubare Polymer ein Molekulargewicht von >5.000 aufweist, wie etwa ein Molekulargewicht von 7.000-17.000, 24.000-38.000, 38.000-54.000, 54.000-69.000 oder 76.000-116.000.

23. Pflaster-Zusammensetzung zur Verwendung nach Anspruch 21 oder 22, wobei das Verhältnis von Lactid zu Glykolid zwischen 85:15 und 50:50 liegt, wie etwa 85:15, 82:18, 75:25, 65:35 oder 50:50.

24. Pflaster-Zusammensetzung zur Verwendung nach einem der Ansprüche 21 bis 23, wobei das wasserlösliche Polymer ausgewählt ist aus der Gruppe bestehend aus Polyvinylpyrrolidon, einem Zucker, wie etwa Saccharose oder Trehalose, Dextran, Carboxymethylcellulose und Natriumalginat.

25. Pflaster-Zusammensetzung zur Verwendung nach Anspruch 24, wobei das wasserlösliche Polymer Polyvinylpyrrolidon ist.

26. Pflaster-Zusammensetzung zur Verwendung nach einem der Ansprüche 21 bis 25, wobei der therapeutisch wirksame Inhaltsstoff ausgewählt ist aus der Gruppe bestehend aus einem Vitamin D-Analogon, einem Glukokortikoid-Rezeptor-Modulator, Ingenol oder einem Ingenolderivat, einem Calcineurinhemmer, einem JAK-Hemmer, einem PDE4-Hemmer, einer nicht steroidalen entzündungshemmenden Substanz, einem Antibiotikum, einem Antimykotikum oder einem Lokalanästhetikum oder Gemischen davon.

27. Pflaster-Zusammensetzung zur Verwendung nach einem der Ansprüche 21 bis 26, wobei der Hautzustand Psoriasis, Strahlenkeratose, Plattenepithelkarzinom, Basalzellkarzinom, Kontaktdermatitis, atopische Dermatitis, ein Ekzem, ein Handekzem, Warzen, Genitalwarzen, Alopezie, Akne, Rosazea oder Hautentzündungen ist.

## Revendications

1. Composition de timbre à micro-aiguilles comprenant une ou plusieurs micro-aiguilles comprenant chacune :
(a) une portion pointe effilée contenant un ingrédient thérapeutiquement actif dispersé dans une matrice d'un polymère biodégradable capable d'assurer une libération prolongée de l'ingrédient thérapeutiquement actif pendant une période d'au moins deux jours après l'insertion de la micro-aiguille ou des micro-aiguilles dans la peau, le polymère biodégradable étant l'acide polylactique ou un polylactide à terminaison ester, l'acide polyglycolique ou un polyglycolide à terminaison ester, ou l'acide polylactique-co-glycolique ou un polylactide-co-glycolide à terminaison ester, et
(b) une portion couche de support des micro-aiguilles, à dissolution rapide, contenant un polymère soluble dans l'eau recouvrant la partie pointe,
ladite micro-aiguille ou lesdites micro-aiguilles étant attachées à et s'étendant à partir d'une surface adhésive d'un substrat amovible,
l'ingrédient thérapeutiquement actif étant libéré à partir des micro-aiguilles pendant une période de 2 à 14 jours après l'insertion de la micro-aiguille ou des micro-aiguilles dans la peau.

2. Composition de timbre selon la revendication 1, comprenant 2 à 100 microaiguilles par cm², p. ex. 5 à 75 microaiguilles, 10 à 50 micro-aiguilles, 15 à 30 microaiguilles ou 20 à 25 microaiguilles par cm².

3. Composition de timbre selon l'une quelconque des revendications 1 à 2, le polymère biodégradable ayant un poids moléculaire de > 5 000, tel qu'un poids moléculaire de 7 000 à 17 000, 24 000 à 38 000, 38 000 à 54 000, 54 000 à 69 000 ou 76 000 à 116 000.

4. Composition de timbre selon la revendication 2 ou 3, le rapport de lactide à glycolide étant compris entre 85:15 et 50:50, tel que 85:15, 82:18, 75:25, 65:35 ou 50:50.

5. Composition de timbre selon l'une quelconque des revendications 1 à 4, la matrice de polymère biodégradable comprenant en outre un antioxydant, p. ex. du butylhydroxytoluène, du butylhydroxyanisole ou de l'a-tocophérol, ou un mélange de ceux-ci.

6. Composition de timbre selon l'une quelconque des revendications 1 à 5, le polymère soluble dans l'eau étant sélectionné dans le groupe constitué par la polyvinylpyrrolidone, un sucre tel que le sucrose ou le tréhalose, le dextrane, la carboxyméthylcellulose et l'alginate de sodium.

7. Composition de timbre selon la revendication 6, le polymère soluble dans l'eau étant la polyvinylpyrrolidone.

8. Composition de timbre selon l'une quelconque des revendications 1 à 7, la portion couche de support comprenant un plastifiant, p. ex du glycérol, du polyéthylène glycol, du sébacate de dibutyle, du phtalate de diéthyle, de la triéthylglycérine ou du citrate de triéthyle.

9. Composition de timbre selon l'une quelconque des revendications 1 à 8, la portion couche de support recouvrant la base de la portion pointe de telle manière que chaque micro-aiguille est séparée des autres micro-aiguilles sur le timbre et forme une entité discrète lorsque le substrat est retiré lors de l'application du timbre sur la peau.

10. Composition de timbre selon l'une quelconque des revendications 1 à 9, les micro-aiguilles étant soit coniques, soit pyramidales, comprenant ainsi un nombre de crêtes s'étendant longitudinalement afin de faciliter l'insertion des microaiguilles dans la peau.

11. Composition de timbre selon l'une quelconque des revendications 1 à 10, l'ingrédient thérapeutiquement actif étant sélectionné dans le groupe constitué par un analogue de vitamine D, un modulateur du récepteur des glucocorticoïdes, l'ingénol ou un dérivé d'ingénol, un inhibiteur de calcineurine, un inhibiteur de JAK, un inhibiteur de PDE4, un agent antiinflammatoire non stéroïdien, un antibiotique, un agent antifongique ou un anesthésique local, ou des mélanges de ceux-ci.

12. Composition de timbre selon l'une quelconque des revendications 1 à 11, comprenant une ou plusieurs micro-aiguilles comprenant chacune :
(a) une portion pointe effilée contenant un ou plusieurs ingrédients thérapeutiquement actifs sélectionnés dans le groupe constitué par le calcipotriol er un ester de bétaméthasone dispersés dans une matrice d'un polymère biodégradable sélectionné dans le groupe constitué par un polylactide à terminaison ester, un polyglycolide à terminaison ester et un polylactide-co-glycolide à terminaison ester, et
(b) une portion couche de support des micro-aiguilles, à dissolution rapide, contenant un polymère soluble dans l'eau recouvrant la portion pointe,
ladite micro-aiguille ou lesdites micro-aiguilles étant attachées à et s'étendant à partir d'une surface adhésive d'un substrat amovible.

13. Composition de timbre selon la revendication 12, l'ester de bétaméthasone étant le dipropionate de bétaméthasone ou le valérate de bétaméthasone, en particulier le dipropionate de bétaméthasone.

14. Composition de timbre selon la revendication 12 ou 13, comprenant 0,08 à 30 µg de calcipotriol par cm².

15. Composition de timbre selon l'une quelconque des revendications 12 à 14, comprenant 1 à 60 µg de dipropionate de bétaméthasone par cm².

16. Composition de timbre selon l'une quelconque des revendications 12 à 15, le polymère biodégradable étant un polylactide-co-glycolide à terminaison ester.

17. Composition de timbre selon l'une quelconque des revendications 12 à 16, le polymère biodégradable ayant un poids moléculaire > 5 000, tel qu'un poids moléculaire de 7 000 à 17 000, 24 000 à 38 000, 38 000 à 54 000, 54 000 à 69 000 ou 76 000 à 116 000.

18. Composition de timbre selon la revendication 16 ou 17, le rapport de lactide à glycolide étant compris entre 85:15 et 50:50, tel que 85:15, 82:18, 75:25, 65:35 ou 50:50.

19. Composition de timbre selon l'une quelconque des revendications 12 à 18, la couche de support comprenant du calcipotriol et/ou un ester de bétaméthasone dispersés dans la matrice du polymère soluble dans l'eau.

20. Composition de timbre selon l'une quelconque des revendications 12 à 19, le polymère soluble dans l'eau étant la polyvinylpyrrolidone.

21. Composition de timbre à micro-aiguilles pour une utilisation dans le traitement d'un trouble cutané, la composition comprenant une ou plusieurs micro-aiguilles comprenant chacune :
(a) une portion pointe effilée contenant un ingrédient thérapeutiquement actif dispersé dans une matrice d'un polymère biodégradable capable d'assurer une libération prolongée de l'ingrédient thérapeutiquement actif pendant une période d'au moins deux jours après l'insertion de la micro-aiguille ou des micro-aiguilles dans la peau, le polymère biodégradable étant l'acide polylactique ou un polylactide à terminaison ester, l'acide polyglycolique ou un polyglycolide à terminaison ester, ou l'acide polylactique-co-glycolique ou un polylactide-co-glycolide à terminaison ester, et
(b) une portion couche de support des micro-aiguilles, à dissolution rapide, contenant un polymère soluble dans l'eau recouvrant la partie pointe,
ladite micro-aiguille ou lesdites micro-aiguilles étant attachées à et s'étendant à partir d'une surface adhésive d'un substrat amovible,
l'ingrédient thérapeutiquement actif étant libéré à partir des micro-aiguilles pendant une période de 2 à 14 jours après l'insertion de la micro-aiguille ou des micro-aiguilles dans la peau.

22. Composition de timbre pour une utilisation selon la revendication 21, le polymère biodégradable ayant un poids moléculaire de > 5 000, tel qu'un poids moléculaire de 7 000 à 17 000, 24 000 à 38 000, 38 000 à 54 000, 54 000 à 69 000 ou 76 000 à 116 000.

23. Composition de timbre pour une utilisation selon la revendication 21 ou 22, le rapport de lactide à glycolide étant compris entre 85:15 et 50:50, tel que 85:15, 82:18, 75:25, 65:35 ou 50:50.

24. Composition de timbre pour une utilisation selon l'une quelconque des revendications 21 à 23, le polymère soluble dans l'eau étant sélectionné dans le groupe constitué par la polyvinylpyrrolidone, un sucre tel que le sucrose ou le tréhalose, le dextrane, la carboxyméthylcellulose et l'alginate de sodium.

25. Composition de timbre pour une utilisation selon la revendication 24, le polymère soluble dans l'eau étant la polyvinylpyrrolidone.

26. Composition de timbre pour une utilisation selon l'une quelconque des revendications 21 à 25, l'ingrédient thérapeutiquement actif étant sélectionné dans le groupe constitué par un analogue de vitamine D, un modulateur du récepteur des glucocorticoïdes, l'ingénol ou un dérivé d'ingénol, un inhibiteur de calcineurine, un inhibiteur de JAK, un inhibiteur de PDE4, un agent antiinflammatoire non stéroïdien, un antibiotique, un agent antifongique ou un anesthésique local, ou des mélanges de ceux-ci.

27. Composition de timbre pour une utilisation selon l'une quelconque des revendications 21 à 26, le trouble cutané étant le psoriasis, la kératose actinique, le carcinome des cellules squameuses, le carcinome des cellules basales, la dermatite de contact, la dermatite atopique, l'eczéma, l'eczéma des mains, les verrues, les verrues génitales, l'alopécie, l'acné, la rosacée ou les infections cutanées.
